# EUROPÄISCHE PATENTSCHRIFT

(11) **EP 2 328 518 B1**
(45) Veröffentlichungstag und Bekanntmachung des Hinweises auf die Patenterteilung: **27.04.2016**
(21) Anmeldenummer: 09776116.7
(22) Anmeldetag: 27.08.2009
(51) Int. Cl.: A61F 2/30, A61F 2/38, A61B 17/72

(54) **KNIEARTHRODESE- IMPLANTAT**
KNEE ARTHRODESIS IMPLANT
IMPLANT POUR ARTHRODÈSE DU GENOU

(30) Priorität: 02.09.2008 DE 102008045291
(43) Veröffentlichungstag der Anmeldung: 08.06.2011
(73) Patentinhaber: Merete Medical GmbH, 12247 Berlin (DE)
(72) Erfinder: ANAPLIOTIS, Emmanuel, 14195 Berlin (DE); KRANZ, Curt, 14163 Berlin (DE); HILSE, Martin, 12105 Berlin (DE); LOB, Guenter, 81377 München (DE)
(74) Vertreter: Hannig, Wolf-Dieter
(86) Internationale Anmeldenummer: PCT/DE2009/001194
(87) Internationale Veröffentlichungsnummer: WO 2010/025704

(56) Entgegenhaltungen:
- EP-A- 1 529 493
- DE-A1- 19 722 389
- DE-U1- 8 306 663
- DE-U1- 8 903 850
- DE-U1-202004 015 578
- US-A- 4 938 768

## Beschreibung

Die Erfindung betrifft ein Kniearthrodese-Implantat mit einem durch in den Knochenmarkkanal des Femur eingeführten Femurnagel und einem in den Knochenmarkkanal der Tibia eingeführten Tibianagel, die miteinander durch ein Verbindungmittel starr verbunden sind.

Aus der EP 1 529 493 B1 ist eine sogenannte implantierbare Orthese für die Kniearthrodese gemäß dem Oberbegriff des Anspruchs 1 bekannt, welche ein Femur-Implantat, das einen femoralen Schaft aufweist, der dazu angepasst ist, in den Knochenmarkkanal des Oberschenkelknochens eingeführt zu werden, und ein Tibia-Implantat umfasst, dass einen tibialen Schaft aufweist, der dazu angepasst ist, in den Knochenmarkkanal des Schienbeins eingeführt zu werden, wobei das Femur-Implantat ein proximales Befestigungsende aufweist, das dazu angepasst ist, mit einem dem Tibia-Implantat zugehörigen proximalen Befestigungsende derart zusammenwirken zu können, dass eine starre Befestigung der beiden Implantate zueinander entsteht, wodurch zwischen Schienbein und Oberschenkelknochen und um die proximalen Befestigungsknochen herum eine umlaufende Knochenrekonstruktion ermöglicht wird.
Dieser bekannte Stand der Technik hat den Nachteil, dass für die Fixierung des femoralen Schafts nur eine einzige Verschraubungsmöglichkeit im femoralen Verankerungskopf vorgesehen ist, die den Schaft gegen eine unerwünschte axiale Verschiebung sichern soll. Des Weiteren ist nachteilig, dass der auf dem tibialen Verankerungskopf aufsitzende femorale Verankerungskopf nur durch eine einzige kegelstumpfartige Schraube gesichert ist, die einen Presssitz zwischen femoralen und tibialen Verankerungskopf erzeugt. Unter Operationsbedingungen ist ein solcher Presssitz nur mit Schwierigkeiten erzeugbar.
Von Nachteil ist weiterhin, dass die bekannte Orthese aus relativ vielen Teilen zusammengesetzt ist und damit einen entsprechend großen Abstand zwischen den zu verbindenden Knochenfragmenten erfordert, was eine unerwünschte Extension von Weich-, Muskel- und Sehnenteilen mit entsprechender Traumatisierung nach sich zieht. Außerdem wird die Modularität erschwert.

Ferner ist aus der DE 197 22 389 A1 ein modulares Knie-Arthrodeseimplantat bekannt, dass ein Femurstielteil, mit dem ein Fermurteil über eine konische Klemmverbindung koppelbar ist, und ein Tibiastielteil umfasst, mit dem ein Tibiateil über eine konische Klemmverbindung koppelbar ist, bei der das Femurteil und Tibiateil mittels einer Schwalbenschanzverbindung miteinander starr verbindbar sind. Ein grundsätzlicher Nachteil dieses bekannten Knie-Arthrodeseimplantat besteht darin, dass zwei konische Klemmverbindungen notwendig sind, die jeweils durch separate Spannschrauben gegen axiales Verschieben gesichert werden müssen. Im Übrigen mag zwar eine Schwalbenschwanzverbindung eine starre Verbindung im montierten Zustand sicherstellen, jedoch können bei einer Revision einer solchen Verbindung, beispielsweise infolge Lockerung, Materialermüdung, Verankerung im Knochen, infektionösen Komplikationen usw., während der Operation erhebliche Probleme durch die gegebenenfalls notwenige laterale Verschiebung von Tibia- und Femurstielteil verursacht werden, um Teile auszuwechseln. Ineinander verkeilte Teile erfordern außerdem das Aufbringen großer Kräfte zur Lösung der Teile, was regelmäßig unerwünscht ist.

Aus der DE 83 06 663 U1 ist des Weiteren ein Implantat zur Überbrückung knöcherner Defekte im Bereich des Kniegelenks bekannt, bei dem der Femur- und Tibiaschaft als zwei starr miteinander verbindbare Schaftteile ausgebildet sind, von denen das erste auf einen konischen Zapfen am zweiten Teil steckbar ist.

Allen diesen bekannten Lösungen ist der Nachteil gemeinsam, dass ihre Längen und Winkellagen nicht einstellbar sind und dadurch die Modularität erheblich eingeschränkt ist.

### Aufgabenstellung

Bei diesem Stand der Technik liegt der Erfindung die Aufgabe zugrunde, ein Knie-Arthrodese-Implantat anzugeben, das die Modularität des Implantats durch eine vergrößerte Längenvariabilität erhöht und gleichzeitig die Teileanzahl reduziert sowie Revisionsoperationen vereinfacht.

Diese Aufgabe wird durch ein Kniearthrodese-Implantat eingangs genannter Gattung mit den Merkmalen des Anspruches 1 gelöst.

Vorteilhafte Ausgestaltungen des Kniearthtrodese-Implantats sind den Unteransprüchen entnehmbar.

Die erfindungsgemäße Lösung zeichnet sich dadurch aus, dass ein modulares System zur Überbrückung großflächiger Knochendefekte als Folge einer oder mehrerer fehlgeschlagener Kniearthrodeseimplantation(en) zur Verfügung gestellt werden kann, welches eine hohe Variationsvielfalt bei geringer Teileanzahl besitzt.

Die Übertragung der physiologischen Maximalbelastungen, insbesondere Kräfte und Momente, von Femur auf Tibia gelingt mit besonderem Vorteil dadurch, dass das Verbindungsmittel zwischen Femurnagel und Tibianagel mindestens ein in Achsrichtung der Nägel gelegenes manschettenförmiges Modul mit mindestens zwei in Achsrichtung geteilte, lösbar miteinander verbundene Elemente umfasst, die miteinander eine sich axial erstreckende Durchführung zum Einführen eines Nagelschaftes in die proximalen und distalen Enden der Durchführung ausbilden und die koaxial in Längsrichtung des Moduls angeordnete, jeweils senkrecht zur Achse positionierte, zueinander korrespondierende Ausnehmungen zur Aufnahme eines Spannmittels aufweisen, das im gespannten Zustand den jeweiligen Nagelhals ausschließlich mittels Reibschluss spannbackenartig mit einem ausreichenden Anzugsmoment in der Durchführung fixiert.
Dies stellt sicher, dass eine physiologische Krafteinleitung in den Femur und in die Tibia erfolgen kann.

Die Erfindung geht von der Erkenntnis aus, dass ein am Nagelschaft des Femur- und Tibianagels spannbackenartig angreifendes manschettenartiges Modul einen linienförmig entlang des Umfangs des Nagelhalses verlaufenden Reibschluss zwischen Nagelhals und Durchführung erzeugt, der auch bei größerer Belastung eine axiale Verschiebung und/oder Rotationsbewegung der an der Verbindung beteiligten Teile sicher ausschließt. Die linienhafte Berührung schließt ein Fretting an den Kontaktstellen dadurch aus, dass Hin- und Herbewegungen unterbunden werden. Der linienartig wirkende Reibschluss wird durch eine auf der Innenwandung der Durchführung vorgesehene Profilierung erreicht, wobei der Halsdurchmesser von Femurnagel und Tibianagel auf den Innendurchmesser der Durchführung abgestimmt ist.

Mit den Spannmitteln, die in Längsrichtung des Moduls unmittelbar nebeneinander angeordnet sind, kann der Reibschluss durch ein entsprechend hohes Anzugsmoment gesichert werden. Ein hinreichend großes Anzugsmoment wird beim Einschrauben der Inbusschrauben in die mit Innengewinde versehene korrespondierende Ausnehmung mit einem Drehmomentschlüssel erzeugt.

Von besonderen Vorteil ist weiterhin, dass die spannbackenartige Verbindung zwischen dem Schaft des Femur- oder Tibianagel und dem manschettenartigen Modul durch seine halbschalige Ausbildung auch bei einer späteren Revision problemlos gelöst und die Traumatisierung des Körpergewebes im Bereich der Arthrodese sehr gering gehalten werden kann. Der operative Eingriff wird dadurch sehr vereinfacht, weil alle Spannmittel ventral zugänglich sind.

Durch die Längenvariabilität des Moduls ist das erfindungsgemäße Implantat an die verschiedenartigen Bedingungen der einzelnen Patienten gut anpassbar.
Besondere Vorteile sind insbesondere dann erreichbar, wenn Module unterschiedlicher oder gleicher Länge durch einen Modulverbinder miteinander verbunden werden, so dass die Längenvariabilität des den Femurnagel und den Tibianagel verbindenden Moduls deutlich vergrößert werden kann.

In einer bevorzugten Ausführungsform der Erfindung ist vorgesehen, dass das Modul eine Abwinklung von dorsal nach ventral in Richtung caudal vorzugsweise zwischen 5 und 15° zum Einstellen der Extension, Flexion und der Valgus/Varus-Stellung des Gelenks aufweist, so dass eine leichte Beugestellung des versteiften Gelenks bzw. auch eine Varus/Valgusstellung des versteiften Gelenks erreicht wird.

In einer weiteren bevorzugten Ausführungsform der Erfindung besitzt eines der Module eine Rosette zum Abstützen bei großen Knochenöffnungen.

In weiterer Ausgestaltung der Erfindung besitzen alle Teile des Implantats, vorzugsweise das Verbindungsmodul eine raue Oberfläche, um das Heranwachsen des Implantats im chirurgisch behandelten Implantatbereich zu verbessern.

In einer weiteren vorteilhaften Weiterbildung der Erfindung ist der Außendurchmesser des Nagelhalses an den Innendurchmesser der Durchführung im Verbindungsmodul angepasst, wobei Nägel mit unterschiedliche Längen und Schaftlängen vorgesehen sind.

Alle Teile des erfindungsgemäßen Kniearthrodese-Implantats bestehen aus einem körperverträglichen und körperbeständigen, vorzugsweise metallischen Werkstoff, beispielsweise aus Titan, Tantal, Niob oder deren Legierungen.

Weitere Vorteile und Einzelheiten ergeben sich aus der nachfolgenden Beschreibung unter Bezugnahme auf die beigefügten Zeichnungen.

### Ausführungsbeispiel

Die Erfindung soll nachstehend an einem Ausführungsbeispiel näher erläutert werden.
Es zeigt
Fig. 1 eine Explosionsdarstellung des erfindungsgemäßen Kniearthrodese-Implantats mit einem abgewinkelten Verbindungsmodul,
Fig. 2 eine perspektivische Ansicht eines abgewinkelten Verbindungsmoduls,
Fig. 3a bis 3e eine Draufsicht auf verschiedene Verbindungsmodule eines Bausatzes des erfindungsgemäßen Kniearthrodese-Implantats
Fig. 4 eine perspektivische Ansicht einer Variante des erfindungsgemäßen Kniearthrodese-Implantats mit Rosette,
Fig. 5 eine Explosionsdarstellung einer weiteren Variante des erfindungsgemäßen Kniearthrodese-Implantats mit einem abgewinkelten Verbindungsmodul und einem weiteren Verbindungsmodul,
Fig. 6 eine Draufsicht auf Arthrodesenägel mit distaler Verriegelung unterschiedlicher Länge und Durchmesser und
Fig. 7 eine Draufsicht auf Arthrodesenägel ohne distaler Verriegelung unterschiedlicher Länge und Durchmesser.

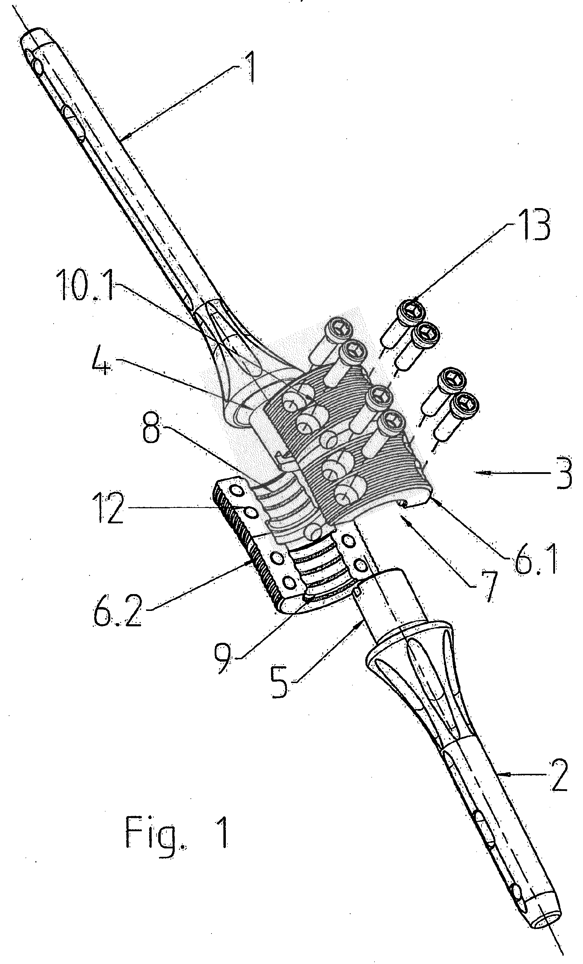

Die Fig. 1 zeigt den grundsätzlichen Aufbau des erfindungsgemäßen Arthrodese-Implantats in Explosionsdarstellung. Das erfindungsgemäße Arthrodese-Implantat, das zur Überbrückung großflächiger Knochendefekte infolge einer fehlgeschlagenen Primärimplanatation oder auch bei der Resektion des metaphysären Bereichs von Femur und Tibia eingesetzt wird, setzt sich aus einem Femurnagel 1, einem Tibianagel 2 und einem Verbindungsmodul 3 zusammen. Der Femurnagel 1 wird in den Knochenmarkkanal der nicht dargestellten Femur und der Tibianagel 2 in den Knochenmarkkanal der nicht dargestellten Tibia eingebracht und verankert. Die Nägel 1 und 2 haben unterschiedliche Längen und Durchmesser und sind anatomisch an die Kontur des Markkanals entsprechend angepasst. Sie sind durch Verriegelungsschrauben bzw. ein sternförmiges Profil ausreichend gegen Rotation im Markkanal gesichert.

Der Femurnagel 1 und der Tibianagel 2 besitzt jeweils einen zylindrischen Nagelhals 4 bzw. 5 aus Vollmaterial. Mit dem Femurnagel 1 ist der Tibianagel 2 mittels des Verbindungsmoduls 3 starr verbunden, das die Nagelhälse 4 bzw. 5 jeweils manschettenförmig umfasst. Das Verbindungsmodul 3 ist zweiteilig und besteht aus zwei zylindrischen Halbschalen 6.1 und 6.2, die im Wesentlichen gleichartig ausgebildet sind. Beide Halbschalen 6.1 und 6.2 definieren im zusammengesetzten Zustand eine Durchführung 7, deren innere Wandung 8 eine senkrecht zur Längsachse LA des Moduls angeordnete Profilierung 9 besitzt. Jeweils vier nebeneinander liegende Ausnehmungen 10.1 und 10.2 sind in jede der Halbschalenwandung 11 so eingebracht, dass die Ausnehmungen koaxial zur Längsachse LA des Moduls 3 und senkrecht zu der von den Halbschalen 6.1 und 6.2 virtuell aufgespannten Teilungsebene TE angeordnet sind, so dass zu jedem Verbindungsmodul 3 mindestens 8 Ausnehmungen gehören. In die Ausnehmungen 10.1 der Halbschale 6.2 ist ein Innengewinde 12 eingebracht, in das eine durch die Ausnehmung 10.1 der Halbschale 6.1 eingeführte Imbusschraube 13 eingeschraubt werden kann.

Die Halbschalen 6.1 und 6.2 bilden mit der jeweiligen Imbusschraube 13 und dem dazu korrespondierenden Innengewinde 12 ein spannbackenartiges Spannmittel 14, das sich beim Anziehen der Imbusschraube 13 um den zylinderförmigen Nagelhals 4 bzw. 5 legt und zwischen der Profilierung 9 und dem Nagelschaft 4 bzw. 5 eine umfangsmäßig umlaufende linienartige Reibschlussverbindung erzeugt. Der Außendurchmesser AD des Nagelschaftes 4 bzw. 5 ist dabei auf den Innendurchmesser ID der Durchführung 7 entsprechend abgestimmt.
Der Widerstand dieser Reibschlussverbindung gegen eine axiale oder eine Rotation der Komponenten lässt sich durch das Aufbringen eines definierten Anzugmomentes auf die Imbusschraube 13 sehr genau und exakt einstellen. Durch die Zuordnung von jeweils vier Spannmitteln 14 am Nagelhals 4 bzw. 5 wird eine sichere Verbindung zwischen dem Femurnagel 1 und dem Tibianagel 2 erreicht.
Alle Spannmittel 14 sind nach ventral ausgerichtet und so bei einer späteren Revision problemlos von außen zugänglich, ohne große Bereiche des körpereigenen Gewebes traumatisieren zu müssen.

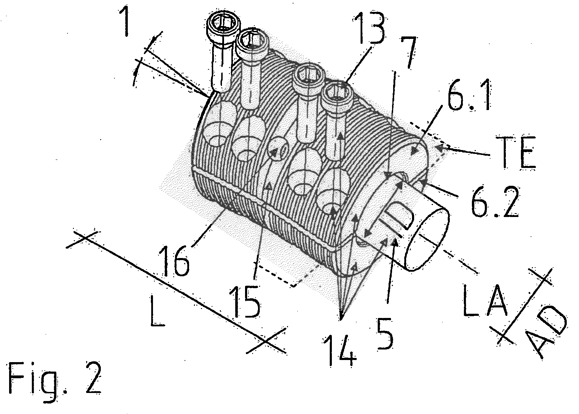

Das Verbindungsmodul 3 besitzt -wie Fig. 2 zeigteine Abwinklung 15, so dass die Extension, Flexion und der Valgus/Varus-Stellung des Gelenks problemlos erreicht werden kann. Die Abwinklung 15 kann in weiten Bereichen, beispielsweise zwischen 1 und 30°, variieren, und ist mit Öffnungen 16 versehen, in die entzündungshemmende oder langzeitwirkende Antibiotika eingebracht werden können.

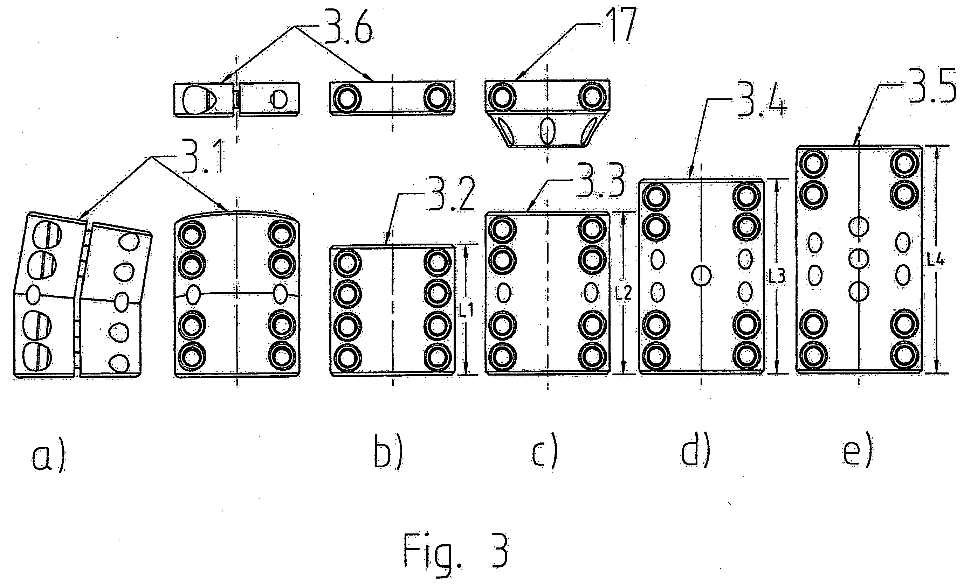

Die Fig. 3a bis 3e zeigt verschiedene Verbindungsmodule 3.1 bis 3.5 mit unterschiedlichen Längen L, beispielsweise ein Verbindungsmodul 3.1 mit Abwinklung 15, ein Verbindungsmodul 3.2 ohne Abwicklung mit jeweils einer Länge L1 (Fig. 3a, 3b), ein Verbindungsmodul 3.3 mit einer Länge L2 (Fig. 3c), L3 (Fig. 3d) und ein Verbindungsmodul 3.4 mit einer Länge L4 (Fig. 3e).
Es versteht sich, dass auch die Verbindungsmodulo 3.3 bis 3.5 jeweils mit einer Abwicklung 15 versehen sein können. Dies wird von den tatsächlichen Gegebenheiten und Bedingungen beim jeweiligen Patienten abhängen.

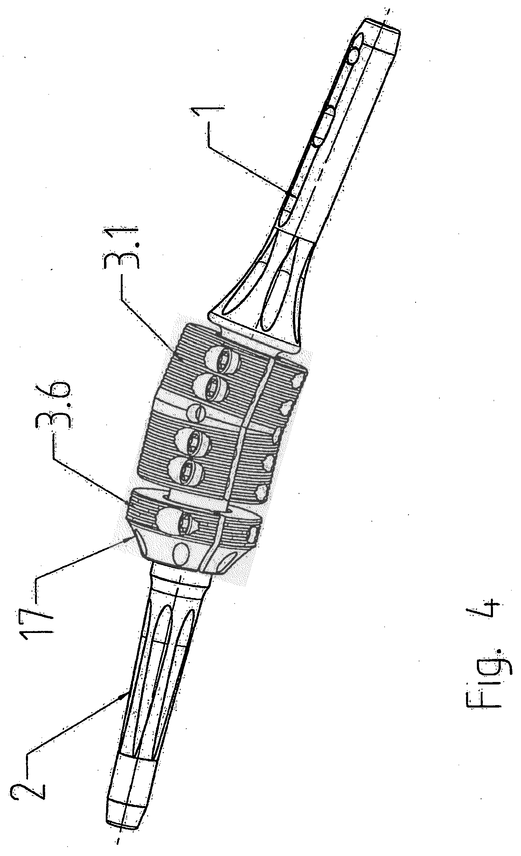

Die Fig. 4 zeigt ein erfindungsgemäßes Kniearthrodese-Implantat, das insbesondere bei großen Knochenöffnungen im Femur besonders geeignet ist. Durch das Verbindungsmodul 3.6 mit einer Länge L von 10 mm ist eine Rosette 17 am Nagelhals 4 des Femurnagel 1 durch Reibschluss fixiert, so dass eine große Knochenöffnung im Knochenmarkkanal des Femur ausgefüllt werden kann. Funktion und Aufbau des Verbindungsmoduls 3.6 mit einer Länge L von 10 mm entspricht der im Abschnitt [0029] aufgeführten Beschreibung für die Verbindungsmodule 3.1 bis 3.5.

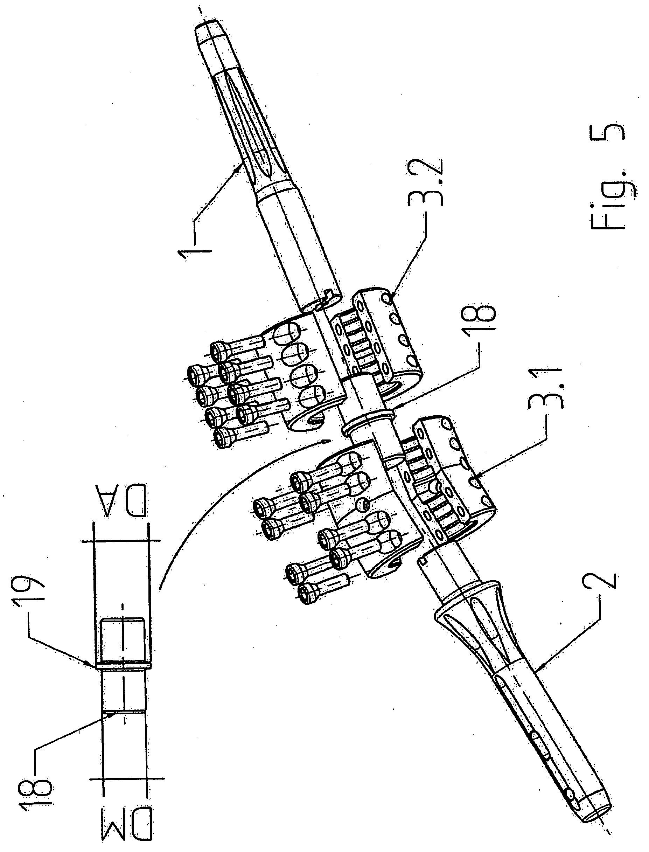

Die Fig. 5 zeigt ein erfindungsgemäßes Kniearthrodese-Implantat, bei dem ein femurseitiges Verbindungsmodul 3.2 und ein tibiaseitiges Verbindungsmodul 3.1 durch einen rundstabförmigen Modulverbinder 18 aneinanderfügbar sind. Der Modulverbinder 18 besteht aus Vollmaterial, dessen Durchmesser DM auf den Innendurchmesser ID der Durchführung 7 abgestimmt ist. Der Modulverbinder 18 weist mittig einen umlaufenden Anschlag 19 mit einem Durchmesser DA auf, der gegenüber dem Innendurchmesser ID der Durchführung 10 größer ist. Durch Einschieben der jeweiligen Enden des Modulverbinders 18 in das entsprechende Ende der Durchführung 7 des Verbindungsmoduls 3.1 und 3.2 und Spannen der jeweilig zugehörenden Spannmittel 14 wird eine Reibschlussverbindung zwischen dem Modulverbinder 18 und den Verbindungsmodulen 3.1 und 3.2 hergestellt.
Dadurch ist es möglich, die einzelnen Verbindungsmodule 3.1 bis 3.6 miteinander zu kombinieren und eine große Längenvariabilität zu erreichen.

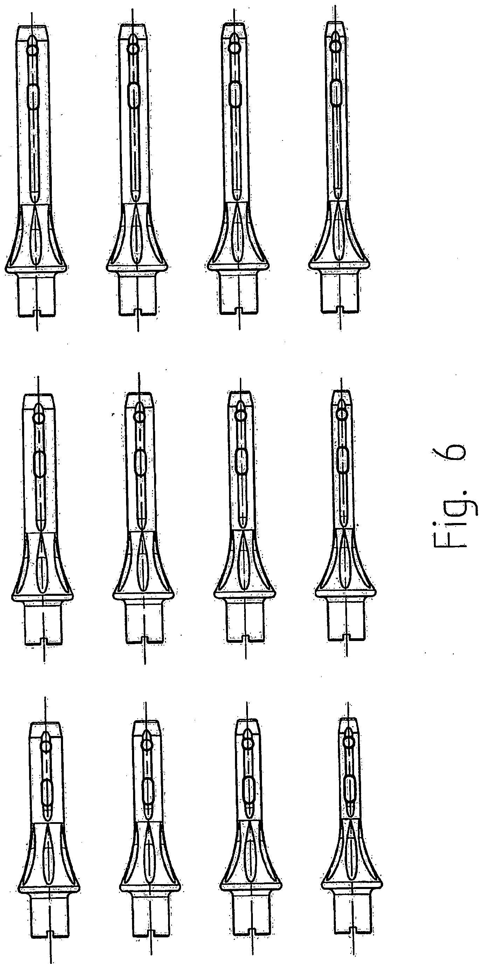

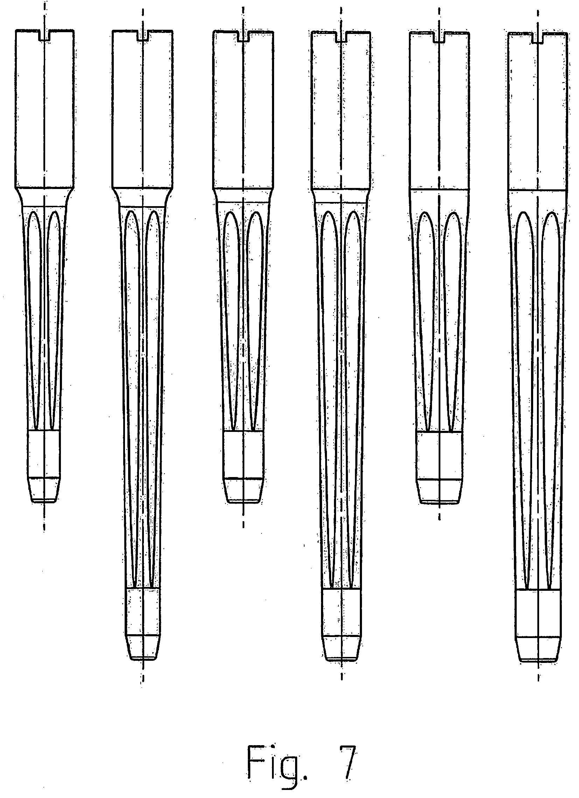

Die Fig. 6 und 7 zeigen Beispiele von Arthrodesenägel mit und ohne distale Verriegelung unterschiedlicher Länge und Durchmesser, deren Halsdurchmesser AD auf den Innendurchmesser ID der Durchführung 7 der jeweiligen Verbindungsmodule 3.1 bis 3.6 abgestimmt sind.

Alle Teile des erfindungsgemäßen Kniearthrodese-Implantats mit Ausnahme der Imbusschrauben bestehen aus einem körperverträglichen und körperbeständigen metallischen Werkstoff, dessen Oberflächen mit einer definierten Rauheit, beispielweise zwischen 20 µm und 80 µm versehen sind. Dies soll einerseits bei den Reibschlusspartnern den Reibkoeffizienten erhöhen und andererseits Einwachsen des Implantats fördern.

Die Erfindung beschränkt sich in ihrer Ausführung nicht auf die vorgenannten Ausführungsbeispiele. Vielmehr sind Varianten denkbar, welche von der dargestellten Lösung auch bei grundsätzlich anders gearteten Ausführungen abweichen können, sofern diese Ausführungen in den Schutzbereich der Patentansprüche fallen.

**Bezugszeichenliste**

| | |
|---|---|
| Femurnagel | 1 |
| Tibianagel | 2 |
| Verbindungsmodul | 3, 3.1-3.6 |
| Nagelhals von 1 | 4 |
| Nagelhals von 2 | 5 |
| Halbschalen | 6.1, 6.2 |
| Durchführung | 7 |
| Innere Wandung von 7 | 8 |
| Profilierung | 9 |
| Ausnehmungen | 10.1, 10.2 |
| Halbschalenwandung | 11 |
| Innengewinde | 12 |
| Imbusschraube | 13 |
| Spannmittel | 14 |
| Abwinklung | 15 |
| Öffnungen in 15 | 16 |
| Rosette | 17 |
| Modulverbinder | 18 |
| Anschlag | 19 |
| Außendurchmesser von 4, 5 | AD |
| Außendurchmesser von 19 | DA |
| Durchmesser von 18 | DM |
| Innendurchmesser von 7 | ID |
| Länge von 3, 3.1-3.6 | L |
| Längsachse von 3, 3.1-3.6 | LA |

## Patentansprüche

1. Kniearthrodese-Implantat mit einem in den Knochenmarkkanal des Femur eingeführten Femurnagel mit einem Nagelhals und einem in den Knochenmarkkanal der Tibia eingeführten Tibianagel mit einem Nagelhals, welche Nägel miteinander durch ein Verbindungmittel starr verbunden sind, das mindestens ein in Achsrichtung der Nägel (1;2) gelegenes manschettenartiges Modul (3,3.1,3.2,3.3,3.4,3.5) aufweist, **dadurch gekennzeichnet, daß** das Modul mindestens zwei in Achsrichtung (LA) des Moduls (3;3.1-3.6) geteilte, Halbschalen (6.1;6.2) umfasst, dass die Halbschalen (6.1; 6.2) lösbar miteinander verbunden sind und eine sich axial erstreckende Durchführung (7) zum Einführen der jeweiligen Nagelhälse (4;5) in die Durchführung (7) ausbilden und daß die Halbschalen koaxial in Achssrichtung des Moduls (3;3.1-3.6) angeordnete, jeweils senkrecht zur Achsrichtung (LA) positionierte, zueinander korrespondierende Ausnehmungen (10.1) zur Aufnahme eines Spannmittels (14) aufweisen, das im gespannten Zustand den jeweiligen Nagelhals (4;5) in der Durchführung (7) ausschließlich mittels Reibschluss spannbackenartig mit einem gegen axiale Verschiebung und Rotation ausreichenden Anzugsmoment fixiert.

2. Kniearthrodese-Implantat nach Anspruch 1, **dadurch gekennzeichnet, dass** das Modul (3;3.1-3.6) im Wesentlichen eine zylindrische Form aufweist und die Halbschalen (6.1;6.2) gleichartig ausgebildet sind.

3. Kniearthrodese-Implantat nach Anspruch 1 und 2, **dadurch gekennzeichnet, dass** das Modul (3:3.1-3.6) längenvariabel ist, wobei Module mit verschiedenen Langen miteinanden kombinierbar sind.

4. Kniearthrodese-Implantat nach Anspruch 1 bis 3, **dadurch gekennzeichnet, dass** das Modul (3;3.1-3.5) eine Abwinkelung (15) zum Einstellen der Extension, Flexion und der Valgus/Varus-Stellung des Gelenks aufweist.

5. Knie-Arthrodese-Implantat nach Anspruch 4, **dadurch gekennzeichnet, dass** die Abwinklung zwischen 1° und 30°, vorzugsweise 5 bis 15°, beträgt.

6. Kniearthrodese-Implantat nach Anspruch 1 bis 3, **dadurch gekennzeichnet, dass** zum Verbinden von Modulen (3;3.1-3.5) gleicher oder unterschiedlicher Länge (L) ein Modulverbinder (18) vorgesehen ist, dessen Außendurchmesser (DM) auf den Innendurchmesser (ID) der Durchführung (7) abgestimmt ist.

7. Kniearthrodese-Implantat nach Anspruch 1, **dadurch gekennzeichnet, dass** die Durchführung (7) an ihrer Innenwandung (8) eine Profilierung (9) zum Verhindern einer Axialbewegung und/oder Fretting von Nagelhals und Modul (3;3.1-3.5) aufweist, die quer zur Längsrichtung (LA) des Moduls (3;3.1-3.5) angeordnet ist.

8. Kniearthrodese-Implantat nach Anspruch 1, **dadurch gekennzeichnet, dass** ein Anschlag- oder Stützmodul (3.6) zum Abstützen einer Rosette (17) am Femurnagel (1) bei großen Knochenöffnungen vorgesehen ist.

9. Kniearthrodese-Implantat nach Anspruch 1 bis 3, **dadurch gekenzennzeichnet**, dass der Durchmesser der Module (3;3.1-3.5) unterschiedlicher Länge (L) gleich ist.

10. Kniearthrodese-Implantat nach Anspruch 1 bis 3, **dadurch gekennzeichnet, dass** der Halsdurchmesser (AD) von Femurnagel (1) und Tibianagel (2) auf den Innendurchmesser (ID) der Durchführung (7) abgestimmt ist.

11. Kniearthrodese-Implantat nach Anspruch 1 bis 10, **dadurch gekennzeichnet, dass** alle Teile des Implantats aus einer biokompatiblen Legierung, vorzugsweise eine Titanlegierung, bestehen.

12. Kniearthrodese-Implantat nach Anspruch 1 bis 11, **dadurch gekennzeichnet, dass** alle Teile des Implantats eine raue Oberfläche zur Förderung des Anwachsens des Knochens aufweist.

13. Kniearthrodese-Implantat nach Anspruch 1, **dadurch gekennzeichnent**, dass das Implantat Spannmittel aufweist und daß die Spannmittel (14) aus einer Imbusschraube (13) und einem in der korrespondierenden Ausnehmung (10.2) vorgesehenen Innengewinde (12) sowie den Halbschalen (6.1; 6.2) bestehen.

14. Kniearthrodese-Implantat nach Anspruch 1 und 13, **dadurch gekennzeichnet, dass** die Schraube (13) durch Senkung und Feingewinde selbsthemmend verklemmt.

15. Kniearthrodese-Implantat nach Anspruch 1 und 13, **dadurch gekennzeichnet, dass** das Spannmittel (14) durch Aufbringen eines bestimmten Anzugsmoments gegen Lösen gesichert ist.

## Claims

1. A knee arthrodesis implant comprising a femur nail, which is introduced into the bone marrow canal of the femur and has a nail neck, and comprising a tibia nail, which is introduced into the bone marrow canal of the tibia and has a nail neck, which nails are connected rigidly together by a connecting means which comprises at least one sleeve-like module (3, 3.1, 3.2, 3.3, 3.4, 3.5) positioned in the axial direction of the nails (1, 2), **characterised in that** the module comprises at least two half-shells (6.1; 6.2) divided in the axial direction (LA) of the module (3; 3.1-3.6), **in that** the half-shells (6.1; 6.2) are connected detachably together and form an axially extending passage (7) for insertion of the respective nail neck (4; 5) into the passage (7) and **in that** the half-shells comprise mutually corresponding openings (10.1) for accommodating a clamping means (14), said openings (10.1) being arranged coaxially in the axial direction of the module (3; 3.1-3.6) and each positioned perpendicular to the axial direction (LA), which clamping means (14), in the clamped state, immobilises the respective nail neck (4; 5) in the passage (7) solely by means of frictional engagement in the manner of clamping jaws with a tightening torque sufficient to counteract axial displacement and rotation.

2. A knee arthrodesis implant according to claim 1, **characterised in that** the module (3; 3.1-3.6) is substantially cylindrical in form and the half-shells (6.1; 6.2) are of similar construction.

3. A knee arthrodesis implant according to claim 1 and claim 2, **characterised in that** the module (3; 3.1-3.6) is variable in length, it being possible to combine together modules of different lengths.

4. A knee arthrodesis implant according to claim 1 to claim 3, **characterised in that** the module (3; 3.1-3.5) is angled (15) to adjust the extension, flexion and valgus/varus position of the joint.

5. A knee arthrodesis implant according to claim 4, **characterised in that** the angle formed is between 1° and 30°, preferably between 5 and 15°.

6. A knee arthrodesis implant according to claim 1 to claim 3, **characterised in that**, to connect modules (3; 3.1-3.5) of identical or different lengths (L), a module connector (18) is provided, the external diameter (DM) of which is matched to the internal diameter (ID) of the passage (7).

7. A knee arthrodesis implant according to claim 1, **characterised in that** the passage (7) comprises profiling (9) on its inner wall (8) to prevent axial movement and/or fretting of nail neck and module (3; 3.1-3.5), which profiling (9) is arranged transversely of the longitudinal direction (LA) of the module (3; 3.1-3.5).

8. A knee arthrodesis implant according to claim 1, **characterised in that** a limit stop or supporting module (3.6) is provided for supporting a round anchor plate (17) on the femur nail (1) in the case of large bone orifices.

9. A knee arthrodesis implant according to claim 1 to claim 3, **characterised in that** the diameter of the modules (3; 3.1-3.5) of different lengths (L) is identical.

10. A knee arthrodesis implant according to claim 1 to claim 3, **characterised in that** the neck diameter (AD) of femur nail (1) and tibia nail (2) is adapted to the internal diameter (ID) of the passage (7).

11. A knee arthrodesis implant according to claim 1 to claim 10, **characterised in that** all the parts of the implant consist of a biocompatible alloy, preferably a titanium alloy.

12. A knee arthrodesis implant according to claim 1 to claim 11, **characterised in that** all the parts of the implant have a rough surface for promoting growth of bone thereon.

13. A knee arthrodesis implant according to claim 1, **characterised in that** the implant has clamping means and **in that** the clamping means (14) consist of a hexagon socket screw (13) and an internal thread (12) provided in the corresponding opening (10.2) as well as of the half-shells (6.1; 6.2).

14. A knee arthrodesis implant according to claim 1 and claim 13, **characterised in that** the screw (13) is self-locking owing to the countersink and fine-pitch thread.

15. A knee arthrodesis implant according to claim 1 and claim 13, **characterised in that** the clamping means (14) is secured against release by application of a given tightening torque.

## Revendications

1. Implant pour arthrodèse du genou, comprenant un clou fémoral introduit dans le canal médullaire du fémur avec un collet de clou et un clou tibial introduit dans le canal médullaire du tibia avec un collet de clou, lesquels clous sont reliés entre eux de manière rigide par un moyen de liaison comportant au moins un module (3, 3.1, 3.2, 3.3, 3.4, 3.5) de type manchon placé dans le sens axial des clous (1, 2), **caractérisé en ce que** le module comprend au moins deux demi-coques (6.1 ; 6.2) divisées dans le sens axial (LA) du module (3 ; 3.1-3.6), **en ce que** les demi-coques (6.1 ; 6.2) sont reliées entre elles de manière amovible et forment un passage (7) s'étendant axialement pour insertion des collets de clou (4; 5) respectifs dans le passage (7) et **en ce que** les demi-coques comportent des évidements (10.1) disposés coaxialement dans le sens axial du module (3 ; 3.1-3.6), positionnés chacun perpendiculairement au sens axial (LA) et correspondant les uns aux autres pour accueillir un moyen de serrage (14) qui, à l'état serré, immobilise le collet de clou (4, 5) respectif dans le passage (7), exclusivement par friction à la manière d'un mors de serrage, avec un couple de serrage suffisant pour empêcher un déplacement axial et une rotation.

2. Implant pour arthrodèse du genou selon la revendication 1, **caractérisé en ce que** le module (3 ; 3.1-3.6) présente une forme sensiblement cylindrique et **en ce que** les demi-coques (6.1 ; 6.2) sont réalisées de manière identique.

3. Implant pour arthrodèse du genou selon la revendication 1 et 2, **caractérisé en ce que** le module (3 ; 3.1-3.6) est de longueur variable, des modules de longueurs différentes pouvant être combinés entre eux.

4. Implant pour arthrodèse du genou selon la revendication 1 à 3, **caractérisé en ce que** le module (3 ; 3.1-3.5) comporte une angulation (15) pour régler l'extension, la flexion et la position valgus/varus de l'articulation.

5. Implant pour arthrodèse du genou selon la revendication 4, **caractérisé en ce que** l'angulation est comprise entre 1° et 30°, de préférence entre 5 et 15°.

6. Implant pour arthrodèse du genou selon la revendication 1 à 3, **caractérisé en ce qu'**il est prévu un raccord de modules (18) servant à assembler des modules de longueur (L) identique ou différente, raccord dont le diamètre extérieur (DM) est adapté au diamètre intérieur (ID) du passage (7).

7. Implant pour arthrodèse du genou selon la revendication 1, **caractérisé en ce que** le passage (7) présente sur sa paroi intérieure (8) un profilage (9) servant à empêcher un déplacement axial et/ou fretting du collet de clou et du module (3 ; 3.1-3.5), lequel profilage est disposé transversalement au sens longitudinal (LA) du module (3 ; 3.1-3.5).

8. Implant pour arthrodèse du genou selon la revendication 1, **caractérisé en ce qu'**il est prévu un module de butée ou de support (3.6) servant à supporter une rosette (17) sur le clou fémoral (1) en cas d'ouverture osseuse importante.

9. Implant pour arthrodèse du genou selon la revendication 1 à 3, **caractérisé en ce que** le diamètre des modules (3 ; 3.1-3.5) de longueur (L) différente est le même.

10. Implant pour arthrodèse du genou selon la revendication 1 à 3, **caractérisé en ce que** le diamètre du collet (AD) du clou fémoral (1) et du clou tibial (2) est adapté au diamètre intérieur (ID) du passage (7).

11. Implant pour arthrodèse du genou selon la revendication 1 à 10, **caractérisé en ce que** toutes les parties de l'implant sont réalisées en un alliage biocompatible, de préférence un alliage à base de titane.

12. Implant pour arthrodèse du genou selon la revendication 1 à 11, **caractérisé en ce que** toutes les parties de l'implant présentent une surface rugueuse pour favoriser la repousse osseuse.

13. Implant pour arthrodèse du genou selon la revendication 1, **caractérisé en ce que** l'implant comprend des moyens de serrage et **en ce que** lesdits moyens de serrage (14) sont composés d'une vis à six pans creux (13) et d'un filetage intérieur (12) prévu dans l'évidement (10.2) correspondant ainsi que des demi-coques (6.1 ; 6.2).

14. Implant pour arthrodèse du genou selon la revendication 1 et 13, **caractérisé en ce que** la vis (13) est à serrage autobloquant grâce au logement conique et au filetage fin.

15. Implant pour arthrodèse du genou selon la revendication 1 et 13, **caractérisé en ce que** le moyen de serrage (14) est protégé contre le desserrage par application d'un couple de serrage déterminé.
